# EUROPEAN PATENT SPECIFICATION

(11) **EP 0 298 684 B1**
(45) Date of publication and mention of the grant of the patent: **21.04.1993**
(21) Application number: 88306079.0
(22) Date of filing: 04.07.1988
(51) Int. Cl.: A61K 7/09

(54) **Crosslinking reagents useful in treating hair**
Vernetzungsmittel zur Haarbehandlung
Agents de réticulation pour le traitement de la chevelure

(30) Priority: 06.07.1987 US 69929
(43) Date of publication of application: 11.01.1989
(73) Proprietor: UNILEVER PLC, London EC4P 4BQ (GB); UNILEVER N.V., 3013 AL Rotterdam (NL)
(72) Inventor: Suita-Mangano, Patricia, Valley Cottage New York 10989 (US)
(74) Representative: Ford, Michael Frederick

(56) References cited:
- DE-B- 1 238 158
- GB-A- 1 049 111
- US-A- 2 351 718
- US-A- 2 933 365
- US-A- 3 071 515
- US-A- 4 251 445

## Description

The present invention relates to permanent hair shaping compositions and methods for using the same.

In the cold waving of hair at room temperature without application of heat, hair strands, which optionally may be rolled on curlers, are moistened with a waving lotion which performs the reducing function of chemically disrupting the disulfide bonds characteristic of keratins. These bonds determine the cohesion of the protein structure and the reduction plasticizes the fiber to render it deformable without elasticity. Specifically, the shaping of human hair has conventionally been carried out by contacting the hair with a reducing agent such as a water-soluble mercaptocarboxylic acid exemplified by ammonium thioglycolate and sulfite/bisulfite exemplified by the sodium salts in the form of liquids or thickened creams and gels. The composition is applied to the hair for a sufficient time to allow shaping by the reductive disruption of the disulfide linkages to form SH groups. The composition is then washed out with water and the hair treated with a fixation or neutralization agent (oxidizing agent) which forms disulfide linkages in new positions causing permanent reshaping of the hair so treated.

If permanent straightening of the hair is desired, the reducing lotion is applied in a thickened form, such as, for instance, in a cream, and evenly distributed throughout the hair by combing. After the composition has acted upon the hair for a sufficient period of time to allow cleavage of the disulfide linkages, during which the hair is combed more or less continuously and maintained in a straightened condition, the shaping agent is washed out with water. The hair is then fixed or set in the known manner, i.e., with a setting composition containing an oxidizing agent.

Rupture of ionic linkages or hydrogen bonds characteristic of a hair set does not provide permanent waving. Cleavage of cystine linkages and their subsequent reformation in a new position after shaping provides permanent deformation. When the disulfide bonds are disrupted in the permanent waving process, the hair fiber is greatly weakened until disulfide bonds are reformed. Thus, when the fiber is subjected to tensile forces, the individual polypeptide chains may be pulled apart since the disulfide bonds which normally hold the polypeptide chains together are no longer present or at least a number of them is reduced.

In U.S. Patent Nos. 2,850,351 and 2,933,365, it is shown that the use of a bis-unsaturated crosslinking reagent wherein the unsaturated groups are activated by an adjacent carbonyl group will react with SH groups to chemically modify keratins, particularly wool. It is also suggested in these patents that the crosslinking reagents disclosed therein are useful for crosslinking between two pendent NH₂ groups. The compounds set forth in the aforementioned patents are limited to operable functions by the requirement of an unsaturated group adjacent to a carbonyl group for the crosslinking function. Treatments for wool can be more extensive than treatment to living hair due to length of time available for treatment and lack of toxicity problems. A wider scope in crosslinking reagents for keratins is therefore desired.

In accordance with the present invention, it has been found that hair, can be chemically modified by the formation of crosslinkages between pendent SH groups and pendent amino groups on the hair using crosslinking reagents having a moiety which is capable of preferentially reacting with a pendent SH group and a moiety which is capable of preferentially reacting with a pendent NH₂ group under the conditions of treatment, the reagent being used to preferentially crosslink between an SH group such as from disrupted disulfide bonds and a pendent NH₂group in the protein molecule. In the preferred embodiments of the invention, the use of these crosslinking reagents in accordance with the present invention has provided hair which is characterized by good curl formation and, in many instances, increased tensile strength. The present invention is particularly adapted for curling treated, e.g., bleached hair.

In a first aspect, therefore, the present invention provides a process for treating hair, as set out in claim 1.

While a wide group of hair types can be treated in accordance with the present invention including animal hair such as camel hair, mohair, wool, horsehair, cattle hair, hog bristles and the like, the invention is particularly directed to waving human hair whether living or detached, i.e., in the form of wigs. The invention is particularly directed to "cold waving" and hair straightening systems; the hair may be Caucasian or ethnic Black.

In a further aspect the invention provides a hair waving system comprising a composition for reducing disulphide bonds in hair, and a crosslinking reagent as set out in claim 1.

The present invention will be discussed, and preferred forms described, in connection with the treatment of human hair to effect deformation to a desired shape. Prior to application of crosslinking reagent, the disulphide linkages have to be disrupted by the action of a reducing agent.

The reducing agents most commonly used in cold waving hair lotions for rupturing cystine linkages are thiols or mercaptans as well as sulfites and/or bisulfites. A number of mercaptans can only provide acceptable efficiency at high pH whereas others with a lower pK and a high ionization constant can be effective at lower pH levels. For example, the ammonium salt of thioglycolic acid can provide acceptable waving efficiency (reduction) if the pH of the solution exceeds 9. Other compounds such as thioglycolamides or glycol thioglycolates, sulfites and/or bisulfites can be used at neutral or even slightly acidic pH. The following are mercaptans and thiols which have commonly been used in cold waving lotions: thioglycolic acid, thiolactic acid, cysteine thioglycerol, thioglycolic hydrazide, thioglycolamide, glycerol monothioglycolate, beta-mercaptopropionic acid, N-hydroxyethyl mercapto-acetamide, N-methyl mercapto-acetamide, beta-mercapto-ethylamine, beta-mercaptopropionamide, 2-mercapto-ethanesulfonic acid, dimercaptoadipic acid, dithiothreitol, homocysteinethiolactone, cysteine derivatives, and polythiol derivatives formed by the addition of cysteamine onto a maleic anhydride-alkylvinylether copolymer. The sulfites and/or bisulfites which can be used are those normally used in hair waving such as the sodium and ammonium salts. The amount of the reducing agent used is that sufficient to rupture a sufficient number of disulfide bonds for effective hair waving or hair straightening as would be appreciated by one ary skill in the art.

By the breaking of the disulfide bonds to form free sulfhydryl groups pendent on the hair, the hair can be formed or shaped as desired such as by winding on rollers or pins, or combed out as in the case of hair straightening. The breaking of the disulfide bonds is generally accomplished in accordance with the usual practice, which involved applying the reducing agent to the hair wound on curlers. Heat can be provided at this point.

The deformed hair, while curled or straightened, is then wetted with a crosslinking composition containing the crosslinking reagent in water or in a carrier or base such as a lotion or cream, preferably buffered. The carriers are of known types and are similar to those presently in use in waving and "neutralizing" compositions. The water or carrier desirably holds the crosslinking reagent in contact with the hair for a period of time sufficient to effect permanent setting of the hair. The crosslinking reagent is applied under conditions conducive to effecting crosslinking. A pH of between about 6 and about 9 (less than that which would cause permanent breakdown of the hair protein) has been found effective for that purpose using the reagents of the invention.

The crosslinking reagents of the present invention can generically be described as having a moiety which is capable of preferentially forming a chemical bond with an SH group joined by means of a divalent organic radical which acts as a joining means with a moiety which is capable of preferentially forming a chemical bond with a pendent NH₂ on the keratin under the conditions of reaction, such as a pH which is substantially neutral to mildly alkaline and not sufficiently alkaline to destroy peptide linkages. Preferably, the crosslinking reagents are water soluble.

As used herein, the term "capable of preferentially forming a chemical bond " is intended to mean that the crosslinking reagent can form a majority of bonds between the groups stated, e.g., between pendent sulfhydryl groups and pendent amino groups and that the bond is a chemical bond that has been developed between the crosslinking reagent and the groups, e.g., an SH group and an NH₂ group though bonds between pendent SH to SH and pendent NH₂ to NH₂ groups can also be formed. The present invention is not limited to the actual bonding sites as all types of bonding may exist at one time. These bonds can be additive such as between ethylenic unsaturation and an SH group, displacement such as a halogen being displaced by the sulfur and by forming a site for bonding such as by hydrolysis. The term "forming a bond" is intended to cover additive reactions where there is no displacement of a radical, displacement reactions where a radical is cleaved from the crosslinking reagent forming a bond through the residue of the crosslinking reagent, and the like, as would be appreciated by one of ordinary skill in the art.

Crosslinking reagents which are capable of preferentially reacting with an amino group and a sulfhydryl group in accordance with the invention can be represented by Formula I:

(I) Y - R - Z

wherein Y represents any radical which is capable of preferentially reacting with the amino groups of the hair under the conditions of treatment, Z represents any radical which is capable of preferentially reacting with sulfhydryl groups of the hair under the conditions of treatment, and R represents a connection means joining Y and Z such as a divalent organic radical connecting Y and Z. Also included within the generic concept of the invention are reagents wherein Y is directly connected to Z. It is understood that the radicals represented by Y and Z can also react with SH and NH₂ groups respectively.

Such Y radicals (generally amino-reacting) include, but are not limited to:
a) hydroxyl radical (-OH)
b) alkoxyl radical (-OR₂)
c) N-succinimidoxy radicals 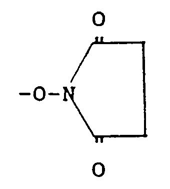
d) acyloxyl radical 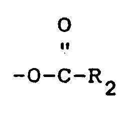
e) haloacidic radicals 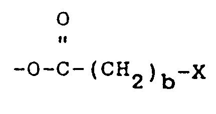 wherein b is an integer, i.e., from about 1 to about 8, preferably 1 to 2 and X represents halogen, preferably where b is 1 and X is iodo and
f) phosphonyl radicals (-O-P(O)₂-R₂)
g) phosphoryl radicals (-O-P(O)₂-O-R₂)
h) sulfonate radicals (-O-S(O)₂-R₂)
i) sulfate radicals (-O-S(O)₂-O-R₂)
   wherein R₂ represents any non-interfering organic substituent such as aryl or alkyl or hydrogen
j) aminobenzoate N-succinimide radicals 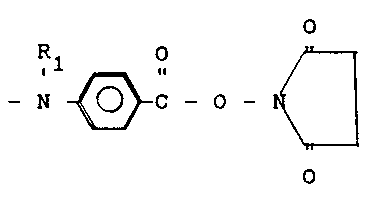 and the like. Other amino-reacting groups may be employed as the Y radical.

Such Z radicals (generally sulfhydryl-reacting) include, but are not limited to, haloacidic radicals [-O-C(O)(CH₂)_{b}-X]. wherein b is an integer and X represents halogen as in e) above, and the corresponding sulfonates as in h) above, sulfates as in i) above, phosphonates as in f) above, phosphates as in g) above and carbonates. The crosslinking with the sulfhydryl group can also occur through an activated olefinic double bond. The olefinic double bond can be catalytically activated by the use of catalysts known by one of ordinary skill in the art for that purpose. Preferably, the olefinic double bond is activated by the presence of a carbonyl group on a carbon atom adjacent to one of the unsaturated carbon atoms. The unsaturated linkage in compounds having two carbonyl groups adjacent to the unsaturated linkage such as derivatives of maleic acid is more greatly activated and are especially preferred. More preferably, the Z radicals are organic groups which contain an acyl nitrogen radical, the acyl radical containing the activated carbon to carbon double bond. The need of extraneous catalysts is avoided by the use of these latter compounds. The ethylenically unsaturated radicals can be derived from compounds illustrated by alkenes (C₂H₂n) such as ethylene propylene, butylene; unsaturated carboxylic acids, esters and amides (R-C=C-C(O)-R₅ wherein R₅ is O- or NH-) such as acrylic acid, methacrylic acid, methyl methacrylate, ethyl methacrylate, acrylamide, methacrylamide; radicals of the formula 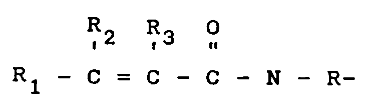
wherein R is as defined hereinbefore, R₁, R₂ and R₃ can independently be hydrogen, lower alkyl, aryl and the like organic groups as long as the substituent is, in general, non-interfering with the crosslinking reaction; R₂ can be -C(O)-R₅ wherein R₅ can be a bond with the amido nitrogen to form a ring; R₃ can be -C-C(O)-R₅ wherein R₅ can be a bond with the amido nitrogen to form a ring. These compounds may be illustrated by imido radicals of maleic acid, citraconic acid, itaconic acid, ethylmaleic acid, alpha-methylitaconic acid, amido radicals of acrylic acid, angelic acid, imides of mono carboxylic unsaturated acids, esters of the aforementioned unsaturated mono and dibasic acids, carbamide derivatives having ethylenic unsaturation such as Rₐ=N-C(O) wherein Rₐ represents the acyl radicals of dibasic unsaturated acids such as maleic, itaconic, citraconic and the like.

The sulfhydryl reacting groups are further illustrated in U.S. Patent No. 2,850,351, the disclosure of which is incorporated herein by reference.

The R radicals include, but are not limited to, any divalent hydrocarbon radical which may be aliphatic, cycloaliphatic or aromatic or a combination of these moieties. Aliphatic includes alkylene (-CₙH₂ₙ-) wherein n can be from 1 to 10 such as methylene; alkylidene (Cₙ₋₁H₂ₙ₋₁CH=) where n can range from 1 to 10 such as ethylidene; aromatic includes phenylene, cyclohexylphenylene, tolylene, xylylene, naphthylene; aminophenylene; cycloaliphatic includes cyclohexylene, methylcyclohexylene, ethylcyclohexylene, and phenyl cyclohexylene. R can also include substituted radicals containing, for example, carboxyl groups, sulphonic groups, amino groups, amido groups, hydroxyl groups and the like, as well as ethers (-Rₓ-O-Rₓ-) and amines (-Rₓ-NH-Rₓ-). Numerous other divalent connecting radicals as would be obvious to one of ordinary skill are included within the representation of R.

Also included within the compounds of Formula I are the halo compounds of Formula II: 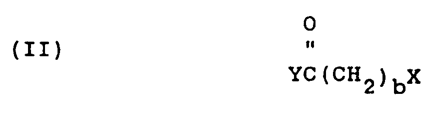
wherein X represents halogen and preferably iodine, and y and b are as defined herein before.

A specific preferred group of these crosslinking compounds is the N-succinimidyl compounds as represented by Formula V hereinafter.

Particularly preferred compounds of the type compounds of Formula I are the compounds based on the succinimide compounds of Formula III: 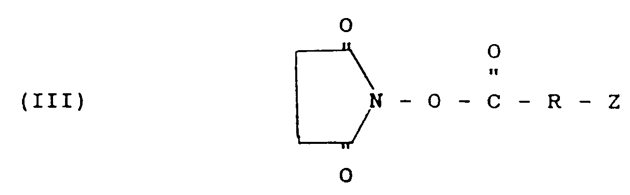
wherein R and Z are as defined in connection with Formula I.

Included within the group of compounds falling within Formula III are the maleimido compounds represented by Formula IV: 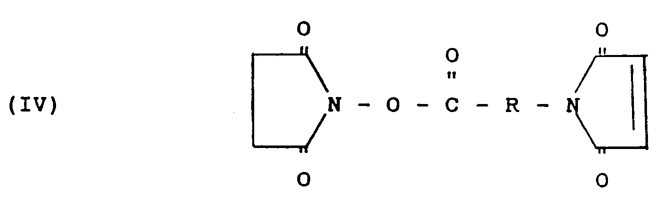
where R is as defined hereinbefore. Preferably, R in Formulae III and IV is phenylene or cyclohexylmethyl (-C₆H₁₀CH₂-).

Also, included within the group of compounds of Formula I are compounds of Formula V: 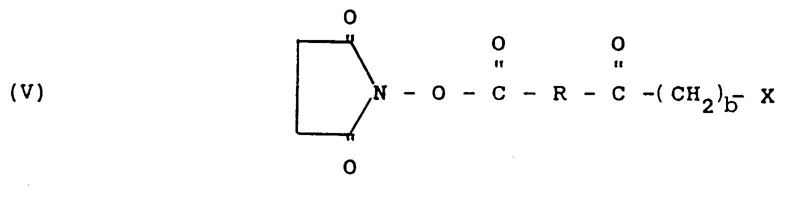
wherein b is as defined previously, and is most preferably 1, X is a halogen and preferably iodine, and R is as defined hereinbefore. More preferably, R is aminophenylene (-C₆H₄-NH-) and the succinimidyl ring is substituted with a water solubilizing group.

It is preferred that the compounds of Formulae I, II, III, IV and V be water soluble and contain a moiety to assist in making the compound water soluble such as a sulfonate group, especially an alkali metal sulfonyl group, attached to R or the succinimidyl ring or quaternary ammonium group such as that normally used in hair waving systems (such as those disclosed in U.S. Patent No. 3,912,808, the disclosure of which is incorporated herein by reference). Preferably, the water solubilization group is attached to any group which is split off in the crosslinking reaction so that the by-product remains water soluble and can be washed out of the hair with water such as the N-succinimidyl radical. The water-solubilizing group can be in the form of a salt such as an alkali metal salt (Na,K) preferably sodium.

The crosslinking reagent is preferably applied to the hair in a buffered system, and particularly a buffered system designed to maintain the pH between about 6 and about 9 and preferably between about 7 and about 8. Any or a combination of alkali metal phosphates, acetates, borates and the like which are non-reactive with the crosslinking reagent to thus destroy crosslinking cites, can be used to maintain the pH of the hair treated with a crosslinking composition within the range specified. To assist in maintaining the pH of the hair within the specified range, it is preferred that the reducing agent not extensively affect the pH of the hair outside the range given. Any pH effects caused by the reducing agent can be offset by thorough washing of the hair with water prior to the application of the crosslinking composition.

The crosslinking reagent is used in an amount sufficient to provide the hair waving and straightening desired. For example, for waving the crosslinking composition can contain an amount of crosslinking reagent ranging from about 0.1% to about 1% weight per volume of buffer. Amounts can vary depending on the effectiveness per unit weight of the crosslinking reagent in crosslinking the hair being treated. The amount of crosslinking composition applied to the hair is generally dictated by the amount needed to wet the hair and not run off. This can vary between users depending on the composition used (such as a gel for hair straightening), the texture of the hair and the tightness of the hair on the roller. Preferred amounts for curling and straightening can be easily determined by one of ordinary skill in the art.

The crosslinking composition can also contain a wetting agent or surfactant which is non-reactive with the crosslinking reagent or the hair to destroy crosslinking cites. The surfactant can be anionic such as soaps and alkyl sulfates such as sodium dodecyl sulfate; cationic such as quaternary ammonium compounds; nonionic such as glycol esters, glycerol esters, sorbitan esters, polyoxyalkalene esters, polyoxyalkylene ethers, and modified lanolin as well as amphoteric surfactants. One of the preferred surfactants is sodium dodecyl sulfate. The surfactant is used in an amount sufficient to assist in wetting the hair with the crosslinking reagent, the amount depending on the efficiency of the surfactant. For example, a sodium dodecyl sulfate can be used in an amount of up to 5% weight per volume of the crosslinking composition. The use and amount of surfactant used is dependent on the origin and treatment history of the hair.

It has also been observed in preliminary testing that virgin (untreated) hair may be more sensitive to the surfactant than bleached hair. Alternatively, the surfactant may find more use in bleached hair than in virgin hair. It was observed that under test conditions, virgin Caucasian hair treated with a crosslinking composition of the invention containing 2% W/V sodium dodecyl sulfate provided good appearance characteristics such as good curl and shiny, but anomalous results on tensile parameters. Virgin Caucasian hair crosslinked without 2% sodium dodecyl sulfate resulted in an increase in tensile parameters. Bleached Caucasian hair treated with the crosslinking composition containing 2% sodium dodecyl sulfate did evidence an increase in tensile parameters. Virgin Black ethnic hair treated with a crosslinking composition containing 0.2% sodium dodecyl sulfate also showed improvement in tensile parameters. The amount of surfactant used may depend on type and treatment history of the hair.

The crosslinking reagents can be applied to the hair alone after reduction or in combination with a reducing agent assuming that the reducing agent is compatible with the crosslinking reagent by techniques similar to those presently used in hair waving to apply waving or oxidizing compositions. The use of the single step system allows for the reduction and immediate crosslinking of the sulfhydryl groups.

The hair is treated for a period of time sufficient to effect the crosslinking to provide the curl and, in some instances, tensile strength increases desired. Illustrative times include from about 3 minutes to any practical, non-deterioration of the hair, upper limit though lesser times can be used if lesser effect on the hair is desired. The hair can be further treated with presently used neutralizing agents and crosslinking reagents in order to oxidize any free SH groups to disulfide linkages as would be appreciated by one of ordinary skill in the art.

The oxidizing or neutralizing chemicals used can be any of the oxidizing agents capable of restoring the disulfide linkages in the hair keratin during the resetting stage, such as aqueous solution of hydrogen peroxide, alkali metal bromates, alkali metal perborates, urea hydrogen peroxide, sodium sesquicarbonate, etc. Rinsing alone with water may restore the broken linkages as well but it will be much slower.

The permanent waving system of the invention can be designed for professional and home application. The system and its compositions can contain ingredients normal to such compositions. Fragrance compounds, coloring agents, thickening agents, opacifying agents, sequestering agents, solubilizing agents, gelling agents, conditioning agents, etc. may be added to compositions of this invention in amounts conventionally used in hair waving and straightening compositions. Any compound which will react with the crosslinking reagent to remove or neutralize reactive cites thereon is preferably avoided. These ingredients are fully outlined in The Science of Hair Care, edited by Charles Zviak, Vol. 7 of a series entitled Dermatology, Marcel Dekker, Inc. 1986 which is incorporated herein by reference.

While the discussion of the invention has related to crosslinking of SH to NH₂ groups, the present application is not intended to be so limited. The invention is intended to cover the use of the compounds of Formulae I, II, III, IV and V and particularly the compounds of Formulae II, III, IV and V as defined hereinbefore as crosslinking reagents in hair regardless of the point of attachment.

It has been specifically found that the compounds N-sulfosuccinimidyl [4-(N-maleimidomethyl)] cyclohexane carboxylate, 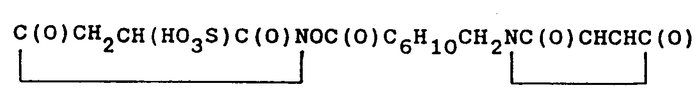
N-sulfosuccinimidyl [3-(N-maleimido)] benzoate 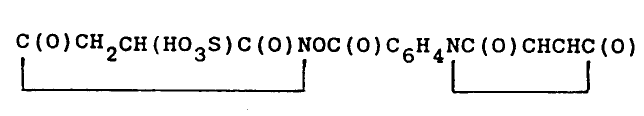
and N-sulfosuccinimidyl (4-iodoacetyl) aminobenzoate 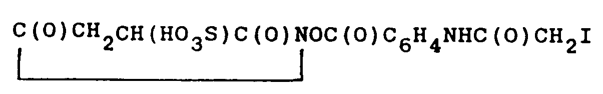
and the sodium salts thereof are effective in treating hair.

As used herein, the terms "halo" and "halogen" are intended to mean chlorine, bromine and iodine unless otherwise stated.

As used herein, the term "% weight per volume" is intended to mean grams per 100 milliliters.

The present invention is more fully illustrated in the Examples which follow.

### EXAMPLE 1

Swatches (0.5 grams; 20.3 centimeters in length) of European brown Caucasian hair wrapped around commercially available curling rods having a central diameter of, for example, 1.25 to 1.6 centimeters were treated with a commercially available waving lotion (Clairol® Professional Kind to Hair Perm System) for 20 minutes to rupture the disulfide bonds. These swatches were then rinsed for 2 minutes with running tap water, treated with 10 milliliters of a crosslinking solution containing about 0.45% weight per volume crosslinking reagents in 50 millimolar of sodium phosphate buffer at pH 7-7.8 and 2% sodium dodecyl sulfate for 10 minutes. The tresses were rinsed again for 2 minutes under running tap water. Some samples were further treated with the neutralizing lotion accompanying the commercial waving lotion in order to oxidize any free SH groups. The crosslinking reagents tested were N-sulfosuccinimidyl [3-(N-maleimido)] benzoate (sulfo-SMB), N-sulfosuccinimidyl [4-(N-maleimidomethyl)) cyclohexane carboxylate (sulfo-SMCC) and N-sulfosuccinimidyl (4-iodoacetyl) aminobenzoate (sulfo-SIAB).

Hair swatches cut to 17.8 centimeters were analyzed for curling length immediately after unrolling the hair swatches, after 2 hours of gentle drying, after 1 hour of soaking in 4 liters of tap water containing a few drops of 29% sodium dodecyl sulfate and after another 2 hours of drying. The lengths were compared during the fifth hour, i.e. the final drying state. Curl lengths comparable to or better than that obtained using the commercial hair waving system (Clairol® Perm System) as control were seen with sulfo-SMB, sulfo-SMCC and sulfo-SIAB. Reoxidation with all three compounds provided curl lengths shorter than the commercial full treatment system. Hair swatches treated with buffer alone, i.e. no crosslinking reagents, for 10 minutes gave very little curl.

The above treated swatches were analyzed for luster, silky feel and combability. All three compounds tested gave appearance results comparable with or better than the commercial lotion treated hair or untreated hair. For example, sulfo-SMB and sulfo-SIAB treated hair gave better looking curls, more luster and more silkiness than the commercially treated hair (Clairol® Perm System) or untreated hair swatches. Sulfo-SMCC treated hair was comparable to the results obtained using the commercial lotion.

### EXAMPLE 2

Swatches as used in Example 1 were treated with another commercial waving lotion (Rave® Performance Perm) for 45 minutes to rupture the disulfide bonds of the hair. These swatches were then rinsed for 2 minutes with running tap water, treated with 5 milliliters of a buffered crosslinking composition containing 0.5% sulfo-SMB (as in Example 1) for 10 minutes and rinsed again for 2 minutes. Some swatches were further treated with the neutralizing lotion accompanying the Rave® Performance Perm for 5 minutes in order to oxidize any free SH groups. Curl lengths were monitored as in Example 1. Shorter curls were obtained utilizing sulfo-SMB as crosslinking reagent before and after reoxidation as compared with a control using a commercial waving lotion. Good luster and softness were obtained with hair swatches treated with the aforementioned crosslinking reagent.

### EXAMPLE 3

### SPLIT-HAIR TECHNIQUE FOR INSTRON TESTING

Each of ten strands of hair for each experimental sample were individually tagged for identification and split in half, one half for treatment, the other for an untreated control. Five of the strands in each half were root to middle with the remaining five strands end to middle. The half was placed in a swatch of hair (as defined in Example 1) and treated without or on a curler. The other half of the hair strands were left untreated as control. The treatments included contacting the hair for 20 minutes with a commercial waving lotion (Clairol® Perm System) to rupture disulfide bonds, rinsed for 2 minutes in flowing tap water, neutralized, i.e. reoxidized, for 5 minutes utilizing the neutralizer accompanying the commercial waving lotion and rinsed for 2 minutes. The second group of hair was treated with a commercial waving lotion (Clairol® Perm System) for 20 minutes to rupture disulfide bonds, rinsed for 2 minutes in flowing tap water, treated with 10 milliliters of a crosslinking solution of sulfo-SIAB in an amount of 0.5% weight per volume in 50 millimolar sodium phosphate buffer at pH 7.8 for 20 minutes, rinsed for 2 minutes in running tap water, neutralized for 5 minutes and rinsed for an additional 2 minutes. A third set was treated the same as in the preceding procedure except that no crosslinking reagent in the buffer was used.

The split hair strands treated with sulfo-SIAB were analyzed for tensile parameters including break point, displacement at break (maximum elongation before break) and total work. The tensile properties were measured on each tagged half strand in an Instron® testing device using the following test conditions:
- Gauge length:: 5.08 cm (2 in.)
- Crosshead speed:: 0.99 cm (0.39 in.)/minute
- Humidity:: 50% relative, or 100% in a water cell
- Temperature:: 22.2°C (72°F)

The following results were obtained:

| Sample | Break Point | Tensile Strength | Elongation to Break | Total Work |
|---|---|---|---|---|
| 50% RH* | + 7% | -- | + 1% | + 11% |
| 100% RH** | -- | + 13% | 0% | + 11% |

| | | | | |
|---|---|---|---|---|
| *RH = Relative Humidity. Results normalized by dividing the result for the treated hair by the result for the virgin hair in the other half of the hair strand. | | | | |
| **RH = Relative Humidity. Split hair technique not used. Hair of the swatch was normalized for cross-sectional area by calculation using mass and density. | | | | |

The tensile properties testing showed a considerable increase in break point (about 7% increase), in tensile strength (about 13% increase) and in total work (about 11% increase) when compared to the untreated hair control.

### EXAMPLE 4

Swatches (as defined in Example 1) of singly-bleached European brown Caucasian hair wherein ten strands were tagged (identified) were treated with Clairol® Perm System as in Example 3. Ten milliliters of a buffered sulfo-SIAB solution containing 0.5% weight sulfo-SIAB per volume (as in Example 1) and 2% sodium dodecyl sulfate was directly applied to the hair wound on rods and then reoxidized as in Example 3. Curl lengths were monitored as in Example 1. Shorter curls were obtained utilizing sulfo-SIAB. Luster, feel and combability comparable to or better than that obtained using the above-mentioned commercial products were obtained with hair swatches treated with the aforementioned crosslinking reagent. The Instron® tensile strength testing showed a 1-8% increase in tensile parameters of hair treated with sulfo-SIAB over control hair treated with a commercial waving system. The percentage range is the range of the results of three separate tests. The improved tensile properties included break point (+5%), elongation to break (+1%) and total work (+8%).

### EXAMPLE 5

Swatches (as defined in Example 1) were treated with Clairol® Perm System as in Example 1. Ten milliliters of a buffered crosslinking composition containing sulfo-SIAB or sulfo-SMB in an amount of 0.5% weight per volume and 2% sodium dodecyl sulfate were directly applied to hair wound on rods and reoxidized as in Example 1. This waving procedure was repeated three times over three consecutive days. Curl lengths were monitored as in Example 1. Curls comparable to or shorter than Clairol® Perm System were obtained on each of the three days. Good luster, feel and combability were obtained with hair swatches treated with sulfo-SIAB or sulfo-SMB.

### EXAMPLE 6

Swatches of Caucasian hair (as defined in Example 1) having included therein ten strands of tagged (identified) ethnic Black hair were treated with Clairol® Perm System as in Example 3 (split hair). Ten milliliters of a buffered solution containing 0.2% sodium dodecyl sulfate and 0.5% weight per volume sulfo-SIAB were directly applied to the hair wound on rods and then reoxidized as in Example 3. The Instron® tests on the isolated ethnic Black hair fibers showed a 11-17% increase in such tensile parameters as break point (+14%), elongation to break (+11%) and total work (+17%) when compared to virgin or Clairol treated ethnic Black hair.

In Black ethnic hair, effective results appear to be obtainable at lower surfactant levels than used for bleached Caucasian hair, e.g., 0.2% vs. 2.0%.

The proportion of Black hair fibers breaking at less than 10% displacement for the fibers crosslinked in accordance with the invention was zero out of 8 whereas 2 fibers out of 9 (22%) waved fibers and 13 out of 69 (19%) virgin fibers broke in the same displacement range.

## Claims

1. A process for treating hair to effect permanent shaping, the disulfide linkages in said hair having been disrupted by the action of a reducing agent forming free sulfhydryl groups, the process comprising contacting the said hair with a crosslinking reagent which has a moiety capable of preferentially forming a bond with a free sulfhydryl group and a moiety which is capable of preferentially forming a bond with a free amino group in the hair, the process being conducted under conditions sufficient to form crosslinkages and insufficient to substantially denature the protein of the hair.

2. The process as recited in claim 1 wherein the crosslinking reagent is a compound of the formula
Y-R-Z
where Y represents any radical which is capable of preferentially reacting with the amino groups of the hair under the conditions of treatment, Z represents any radical which is capable of preferentially reacting with sulfhydryl groups of the keratinous material under the conditions of treatment, and R represents a connection between Y and Z.

3. The process as recited in claim 2 wherein the crosslinking reagent is a compound of the formula 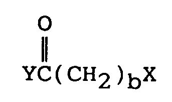 wherein Y is as defined in claim 2, X represents halogen and b is an integer.

4. The process as recited in claim 3 wherein b is 1 and X represents iodo.

5. The process as recited in any one of claims 2 to 4 wherein Y represents a radical selected from the group consisting of hydroxyl, alkoxyl, acyloxyl, haloacidic, N-succinimidyl, phosphonyl, phosphoryl, sulfonate, sulfate and aminobenzoate-N-succinimidyl.

6. The process as recited in claim 3 wherein the crosslinking reagent is a compound of the formula 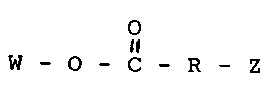 wherein R and Z are as defined in claim 2 and W is an N-succinimidyl group.

7. The process as recited in claim 2 or claim 6 wherein Z represents a radical selected from the group consisting of haloacetyl, other haloacidic, phosphonyl, phosphoryl, sulfonate, sulfate, carbonate, iodine, an activated olefinic double bond and mixtures thereof.

8. The process as recited in claim 7 wherein the activated olefinic double bond is activated by the presence of a carbonyl group on a carbon atom adjacent to one of the unsaturated carbon atoms.

9. The process as recited in claim 8 wherein the radicals containing the activated olefinic double bonds include an acyl nitrogen.

10. The process as recited in claim 7 wherein the haloacetyl is iodoacetyl.

11. The process as recited in claim 2 wherein the crosslinking reagent is a compound of the formula 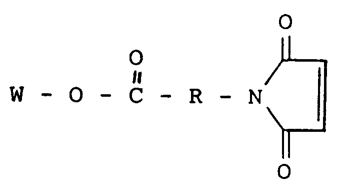 wherein R is as defined in claim 2 and W is an N-succinimidyl group.

12. The process as recited in claim 6 or claim 11 wherein R is a phenylene radical or a -C₆H₁₀-CH₂- radical wherein the -C₆H₁₀ portion is attached to the carbonyl group.

13. The process as recited in claim 2 wherein said crosslinking reagent is a compound of the formula 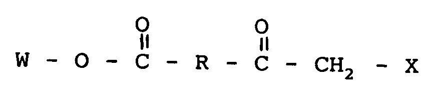 wherein R is as defined as in claim 2, X is halogen and W is an N-succinimidyl group.

14. The process as recited in claim 13 wherein R is -C₆H₄NH- and X is iodo.

15. The process as recited in claim 1 wherein the crosslinking reagent is N-sulfosuccinimidyl [4-(N-maleimidomethyl)] cyclohexane carboxylate, or N-sulfosuccinimidyl (4-iodoacetyl) aminobenzoate, or N-sulfosuccinimidyl [3-(N-maleimido)] benzoate.

16. The process as recited in any one of the preceding claims wherein the crosslinking reagent is substituted with a water solubilizing group.

17. The process as recited in any one of the preceding claims which further includes the step of reoxidizing the keratinous material after formation of crosslinkages.

18. A hair waving system comprising a composition for reducing disulphide bonds in hair and a crosslinking reagent as defined in any one of the preceding claims.

## Patentansprüche

1. Verfahren zur Haarbehandlung, um eine Dauerverformung zu erreichen, wobei die Disulfidbindungen in diesem Haar durch die Wirkung eines freie Sulfhydrylgruppen bildenden Reduktionsmittels aufgetrennt worden sind, wobei das Verfahren das in Kontakt bringen dieses Haares mit einem Vernetzungsmittel umfaßt, das einen Rest aufweist, der in der Lage ist, bevorzugt eine Bindung mit einer freien Sulfhydrylgruppe einzugehen, und einem Rest, der in der Lage ist, bevorzugt eine Bindung mit einer freien Aminogruppe im Haar einzugehen, wobei das Verfahren unter Bedingungen durchgeführt wird, die ausreichend sind, Vernetzungen zu bilden und ungenügend, das Protein des Haares im wesentlichen zu denaturieren.

2. Verfahren nach Anspruch 1, worin das Vernetzungsmittel eine Verbindung der Formel
Y-R-Z
ist, worin Y irgendein Radikal darstellt, das in der Lage ist, unter den Behandlungsbedingungen bevorzugt mit den Aminogruppen des Haars zu reagieren, Z irgendein Radikal darstellt, das in der Lage ist, unter den Behandlungsbedingungen bevorzugt mit Sulfhydrylgruppen des keratinösen Materials zu reagieren, und R eine Verbindung zwischen Y und Z darstellt.

3. Verfahren nach Anspruch 2, worin das Vernetzungsmittel eine Verbindung der Formel 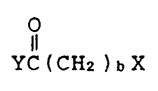 ist, worin Y wie in Anspruch 2 definiert ist, X Halogen bedeutet und b eine ganze Zahl darstellt.

4. Verfahren nach Anspruch 3, worin b für 1 steht und X Jod darstellt.

5. Verfahren nach einem der Ansprüche 2 bis 4, worin Y ein Radikal darstellt, das ausgewählt ist aus der Gruppe Hydroxyl, Alkoxyl, Acyloxyl, Haloacyl, N-Succinimidyl, Phosphonyl, Phosphoryl, Sulfonat, Sulfat und Aminobenzoat-N-succinimidyl.

6. Verfahren nach Anspruch 3, worin das Vernetzungsmittel eine Verbindung der Formel 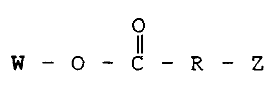 ist, worin R und Z wie in Anspruch 2 definiert sind und W eine N-Succinimidylgruppe darstellt.

7. Verfahren nach Anspruch 2 oder Anspruch 6, worin Z ein Radikal darstellt, das ausgewählt ist aus der Gruppe Haloacetyl, ein anderes Haloacyl, Phosphonyl, Phosphoryl, Sulfonat, Sulfat, Carbonat, Jod, eine aktivierte olefinische Doppelbindung und Mischungen davon.

8. Verfahren nach Anspruch 7, worin die aktivierte olefinische Doppelbindung durch die Anwesenheit einer Carbonylgruppe an einem einem ungesättigten Kohlenstoffatomen benachbarten Kohlenstoffatom aktiviert ist.

9. Verfahren nach Anspruch 8, worin die Radikale, die die aktivierten olefinischen Doppelbindungen enthalten, einen Acyl-Stickstoff enthalten.

10. Verfahren nach Anspruch 7, worin das Haloacetyl Jodacetyl ist.

11. Verfahren nach Anspruch 2, worin das Vernetzungsmittel eine Verbindung der Formel 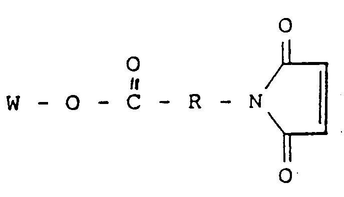 ist, worin R wie in Anspruch 2 definiert ist und W eine N-Succinimidylgruppe darstellt.

12. Verfahren nach Anspruch 6 oder Anspruch 11, worin R ein Phenylenradikal oder ein -C₆H₁₀-CH₂- Radikal bedeutet, worin der -C₆H₁₀- Teil mit der Carbonylgruppe verbunden ist.

13. Verfahren nach Anspruch 2, worin das genannte Vernetzungsmittel eine Verbindung der Formel 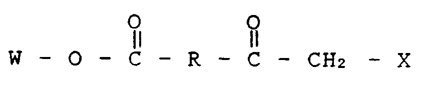 ist, worin R wie in Anspruch 2 definiert ist, X Halogen bedeutet und W eine N-Succinimidylgruppe darstellt.

14. Verfahren nach Anspruch 13, worin R für -C₆H₄NH- steht und X Jod bedeutet.

15. Verfahren nach Anspruch 1, worin das Vernetzungsmittel N-Sulfosuccinimidyl-[4-(N-maleimidomethyl)]-cyclohexancarboxylat oder N-Sulfosuccinimidyl-(4-jodacetyl)-aminobenzoat oder N-Sulfosuccinimidyl-[3-(N-maleimido)]-benzoat ist.

16. Verfahren nach einem der vorhergehenden Ansprüche, worin das Vernetzungsmittel mit einer Wasser-solubilisierenden Gruppe substituiert ist.

17. Verfahren nach einem der vorhergehenden Ansprüche, das zusätzlich die Stufe der Reoxidation des keratinösen Materials nach der Bildung der Vernetzungen umfaßt.

18. Haarwellsystem, enthaltend eine Zusammensetzung zur Reduktion von Disulfidbindungen im Haar und ein Vernetzungsmittel, wie in einem der vorhergehenden Ansprüche definiert.

## Revendications

1. Un procédé de traitement de la chevelure pour effectuer une mise en plis permanente, les liaisons bisulfure de ladite chevelure ayant été perturbées par l'action d'un réducteur formant des groupes sulfydryles libres, le procédé comprenant le fait de mettre en contact ladite chevelure avec un agent de réticulation ayant une partie de molécule capable de former de façon préférentielle une liaison avec un groupe sulfydryle libre, et une partie de molécule capable de former de façon préférentielle une liaison avec un groupe amino libre dans la chevelure, le procédé étant effectué dans des conditions suffisantes pour former des réticulations et insuffisant pour dénaturer de façon substantielle la protéine de la chevelure.

2. Le procédé selon la revendication 1, dans lequel l'agent de réticulation est un composé de formule
Y-R-Z
dans laquelle Y représente tout radical capable de réagir de façon préférentielle avec les groupes amino de la chevelure dans les conditions de traitement, Z représente tout radical capable de réagir de façon préférentielle avec les groupes sulfydryles du matériau de kératine dans les conditions de traitement, et R représente une connexion entre Y et Z.

3. Le procédé selon la revendication 2, dans lequel l'agent de réticulation est un composé de formule 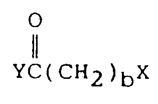 dans laquelle Y est tel que défini dans la revendication 2, X représente de l'halogène et b est un nombre entier.

4. Le procédé selon la revendication 3, dans lequel b est 1 et X représente de l'iode.

5. Le procédé selon l'une des revendications 2 à 4, dans lequel Y représente un radical choisi dans le groupe composé de l'hydroxyle, de l'alkoxyle, de l'acyloxyle, de l'haloacide, du N-succinimidyle, du phosphonyle, de phosphoryle, du sulphonate, du sulphate et de l'aminobenzoate-N-succinimidyle.

6. Le procédé selon la revendication 3, dans lequel l'agent de réticulation est un composé de la formule 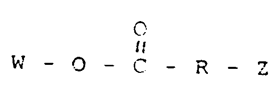 dans laquelle R et Z sont tels que définis dans la revendication 2 et W est un groupe N-succinimidyle.

7. Le procédé selon la revendication 2 ou la revendication 6, dans lequel Z représente un radical sélectionné à partir du groupe composé de l'haloacétyle, d'autres haloacides, du phosphonyle, du phosphoryle, du sulphonate, du sulphate, du carbonate, de l'iode et de la double liaison oléfinique activée et des mélanges de ceux-ci.

8. Le procédé selon la revendication 7, dans lequel la double liaison oléfinique activée est activée par la présence d'un groupe carbonyle sur un atome de carbone en position adjacente par rapport à l'un des atomes de carbone insaturé.

9. Le procédé selon la revendication 8, dans lequel les radicaux contenant les doubles liaisons oléfiniques activées comprennent un azote acyle.

10. Le procédé selon la revendication 7, dans lequel l'haloacétyle est de l'iodoacétyle.

11. Le procédé selon la revendication 2, dans lequel l'agent de réticulation est un composé de formule 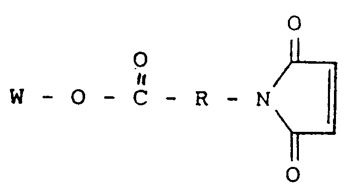 dans laquelle R est tel que défini dans la revendication 2 et W est un groupe N-succinimidyle.

12. Le procédé selon la revendication 6 ou la revendication 11, dans lequel R est un radical phénylène ou un radical en -C₆H₁₀-CH₂- dans lequel la partie -C₆H₁₀ est liée au groupe carbonyle.

13. Le procédé selon la revendication 2, dans lequel ledit agent de réticulation est un composé de formule 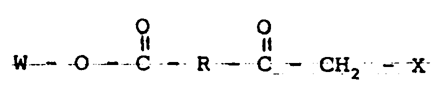 dans laquelle R est tel que défini dans la revendication 2, X est de l'halogène et W est un groupe N-succinimidyle.

14. Le procédé selon la revendication 13, dans lequel R est -C₆H₄HN- et X est iodo.

15. Le procédé selon la revendication 1, dans lequel l'agent de réticulation est du N-sulfosuccinimidyle [4-(N-maléimidométhyl)] cyclohéxane carboxylate, ou du N-sulfosuccinimidyle (4-iodoacétyl) aminobenzoate, ou du N-sulfosuccinimidyle [3-(N-maléimido)] benzoate.

16. Le procédé selon l'une des revendications précédentes, dans lequel l'agent de réticulation est remplacé par un groupe soluble dans l'eau.

17. Le procédé selon l'une des revendications précédentes, comprenant en outre l'étape consistant à réoxyder le matériau de kératine après la formation des réticulations.

18. Un système d'ondulation de la chevelure comprenant une composition permettant de réduire les liaisons bisulfure dans la chevelure et un agent de réticulation tel que défini dans l'une des revendications précédentes.
